# EUROPÄISCHE PATENTSCHRIFT

(11) **EP 3 430 961 B1**
(45) Veröffentlichungstag und Bekanntmachung des Hinweises auf die Patenterteilung: **17.06.2020**
(21) Anmeldenummer: 18183953.1
(22) Anmeldetag: 17.07.2018
(51) Int. Cl.: A47L 7/00, A47L 9/04

(54) **BODENBEARBEITUNGSGERÄT MIT EINEM MOTORISCH ANGETRIEBENEN BODENBEARBEITUNGSELEMENT**
SOIL CULTIVATION DEVICE WITH A MOTORISED DRIVEN SOIL WORKING ELEMENT
APPAREIL DE TRAITEMENT DU SOL POURVU D'UN ÉLÉMENT DE TRAITEMENT DU SOL À ENTRAÎNEMENT PAR MOTEUR

(30) Priorität: 20.07.2017 DE 102017116363
(43) Veröffentlichungstag der Anmeldung: 23.01.2019
(73) Patentinhaber: Vorwerk & Co. Interholding GmbH, 42275 Wuppertal (DE)
(72) Erfinder: Hackert, Georg, 44869 Bochum (DE)
(74) Vertreter: Müller, Enno

(56) Entgegenhaltungen:
- EP-A2- 2 279 686
- EP-B1- 1 505 901
- EP-B1- 2 467 049
- WO-A1-2016/206941

## Beschreibung

### Gebiet der Technik

Die Erfindung betrifft ein Bodenbearbeitungsgerät mit einem motorisch angetriebenen Bodenbearbeitungselement zum Bearbeiten einer zu bearbeitenden Fläche, wobei das Bodenbearbeitungselement einen Hohlraum mit einer den Hohlraum mit der Umgebungsluft verbindenden Hohlraumöffnung aufweist.

### Stand der Technik

Bodenbearbeitungsgeräte mit motorisch angetriebenen Bodenbearbeitungselementen sind im Stand der Technik hinreichend bekannt.

Bodenbearbeitungsgeräte dieser Art können beispielsweise Saugreinigungsgeräte oder Feuchtwischgeräte sein, welche über ein schwingend angetriebenes oder rotierbar angetriebenes Bodenbearbeitungselement verfügen. Beispielsweise kann das Bodenbearbeitungselement eine um eine Drehachse rotierbare Reinigungswalze sein, welche mit einem Reinigungsbelag oder Borstenelementen ausgestattet ist. Ein solches Bodenbearbeitungsgerät zeigt beispielsweise die Druckschrift DE 20 2007 017 026 U1.

Des Weiteren sind auch Bodenbearbeitungsgeräte bekannt, welche keine Reinigungsaufgabe sondern beispielsweise eine Polieraufgabe, Schleifaufgabe oder dergleichen erfüllen. Unter Bodenbearbeitungsgeräten sollen hier des Weiteren auch Geräte verstanden werden, welche über Überbodenflächen reinigen können. Die Bodenbearbeitungsgeräte können entweder von einem Nutzer handgeführte Bodenbearbeitungsgeräte sein, oder beispielsweise auch sich selbsttätig innerhalb einer Umgebung fortbewegende Bodenbearbeitungsgeräte, insbesondere autonome Roboter.

Des Weiteren ist es bekannt, Reinigungsgeräte mit Duftelementen auszustatten, um eine von dem Reinigungsgerät abgegebene Abluft mit Duft zu versehen. Aus der Druckschrift DE 102 09 298 A1 ist es beispielsweise bekannt, einen Filterbeutel für ein Reinigungsgerät auf seiner Abluftseite mit einem Duftelement auszurüsten, das von der aus dem Filterbeutel austretenden Abluft überstrichen wird.

Die bekannten Bodenbearbeitungsgeräte mit Duftelementen sind so beschaffen, dass der von dem Duftelement ausgehende Duft durch von einer Motor-Gebläse-Einheit des Reinigungsgerätes verursachte Abluft in die Umgebung gefördert wird.

Daneben offenbaren die Dokumente EP 1 505 901 B1 und EP 2 467 049 B1 motorisch angetriebene Reinigungs- beziehungsweise Poliergeräte mit einem kissenförmigen Bearbeitungselement, welches mit einem trockenen, flüssigen oder gelförmigen Oberflächenbehandlungsmittel gefüllt ist, das u.a. Parfümstoffe aufweisen kann.

Die Dokumente WO 2016/206941 A1 und EP 2 279 686 A2 offenbaren Reinigungsgeräte mit rotierbaren Bodenbearbeitungselementen, die einen Hohlraum mit einer den Hohlraum mit der Umgebungsluft verbindenden Hohlraumöffnung aufweisen.

### Zusammenfassung der Erfindung

Ausgehend von dem vorgenannten Stand der Technik ist es Aufgabe der Erfindung, ein Bodenbearbeitungsgerät mit einem alternativ angeordneten Duftelement bereitzustellen.

Zur Lösung der vorgenannten Aufgabe wird mit der Erfindung ein Bodenbearbeitungsgerät mit einem motorisch angetriebenen Bodenbearbeitungselement zum Bearbeiten einer zu bearbeitenden Fläche vorgeschlagen, wobei das Bodenbearbeitungselement einen Hohlraum mit einer den Hohlraum mit der Umgebungsluft verbindenden Hohlraumöffnung aufweist, wobei in dem Hohlraum ein als Festkörper ausgebildetes Duftelement angeordnet ist, wobei der Hohlraum ein Volumen aufweist, welches einem Vielfachen des Volumens des Duftelementes entspricht.

Erfindungsgemäß ist das Bodenbearbeitungsgerät nun mit einem Duftelement ausgestattet, welches bei angetriebenem Bodenbearbeitungselement verlagert wird und aufgrund der Verlagerung und der damit verbundenen Luftbewegungen Duftmoleküle durch die Hohlraumöffnung an die Umgebungsluft abgeben kann. Der Duft strömt gemäß einer besonders einfachen Ausführungsform alleine aufgrund der an dem Duftelement vorbeistreichenden, fliehkraftbedingten Luftströme aus dem Hohlraum heraus. Das Duftelement ist vorzugsweise entnehmbar in dem Hohlraum angeordnet, wobei eine Wandung des Hohlraums eine Öffnung aufweist, beispielsweise auch eine Hohlraumöffnung, welche zumindest bezogen auf eine Dimension größer ist als eine Dimension des Duftelementes, so dass dieses aus dem Hohlraum entfernt werden kann. Der Duft des Duftelementes breitet sich insbesondere dann verstärkt über die Umgebungsluft aus, wenn das Bodenbearbeitungselement angetrieben wird, d. h. beispielsweise rotiert wird oder schwingend angetrieben wird. Somit erhält ein Nutzer des Bodenbearbeitungsgerätes, insbesondere bei sich autonom fortbewegendem Bodenbearbeitungsgerät, einen Hinweis darauf, dass das Bodenbearbeitungsgerät eine Bodenbearbeitung, beispielsweise eine Saugreinigung oder Wischreinigung, ausgeführt hat. Der Duft ist somit gleichzeitig ein Signal für die ausgeübte Bodenbearbeitungstätigkeit des Bodenbearbeitungsgerätes. Sofern beispielsweise ein Fehler des motorischen Antriebs des Bodenbearbeitungselementes auftritt, entfällt gleichzeitig das Duftsignal, so dass der Nutzer einen Hinweis auf eine Fehlfunktion erhält.

Gemäß einer bevorzugten Ausführungsform wird vorgeschlagen, dass das Bodenbearbeitungselement eine um eine Drehachse rotierbare Reinigungswalze ist. Die Reinigungswalze kann beispielsweise eine mit einem Reinigungstuch belegte Wischwalze zum Ausführen einer Feuchtreinigungstätigkeit oder auch eine von außen mit Borstenelementen ausgestattete Reinigungswalze für eine Trockenreinigung sein. Das Bodenbearbeitungselement kann insbesondere nach der Art eines Hohlzylinders ausgebildet sein, wobei das Duftelement im Inneren des Zylinders angeordnet ist.

Gemäß einer weiteren Ausführungsform wäre es denkbar, dass das Bodenbearbeitungselement nach der Art einer Platte mit einem innenliegenden Hohlraum ausgestattet ist, wobei die Platte schwingend parallel zu der zu bearbeitenden Fläche angetrieben wird, insbesondere mit einer exzentrischen Kreisbewegung über die zu bearbeitende Fläche geführt wird.

Es wird vorgeschlagen, dass eine Lebensdauer des Duftelementes im Wesentlichen einer Lebensdauer eines Verschleißteils des Bodenbearbeitungsgerätes entspricht. Insbesondere kann die Lebensdauer des Duftelementes im Wesentlichen einer Lebensdauer von an dem Bodenbearbeitungselement angeordneten Borstenelementen entsprechen. Durch diese Ausgestaltung wird eine Verbrauchszeit des Duftelementes an einen Verschleiß eines Verschleißteils des Bodenbearbeitungsgerätes gekoppelt. Bei dem Verschleißteil kann es sich um das Bodenbearbeitungselement selbst, insbesondere an diesem angeordnete Borstenelemente oder ein an diesem angeordnetes Reinigungstuch handeln. Der Nutzer des Bodenbearbeitungsgerätes erhält somit einen Hinweis darüber, wann das Verschleißteil zu ersetzen ist. In dem Fall, dass es sich bei dem Verschleißteil um das Bodenbearbeitungselement handelt, wird gleichzeitig mit dem Bodenbearbeitungselement vorzugsweise auch das darin angeordnete Duftelement ersetzt, so dass ein neu an dem Bodenbearbeitungsgerät angeordnetes Bodenbearbeitungselement gleichzeitig auch wieder Duft abgibt. Der Verbrauch sowohl des Bodenbearbeitungselementes als auch des Duftelementes hängt in erster Linie von der Betriebszeit des Bodenbearbeitungselementes bzw. des Duftelementes ab, so dass diese Zeitspannen vorteilhaft aufeinander abgestimmt werden können.

Des Weiteren wird vorgeschlagen, dass das Material des Duftelementes eine geringere Härte aufweist als das Material einer den Hohlraum begrenzenden Wandung. Insbesondere kann das Duftelement aus einem weichelastischen Material hergestellt sein, besonders bevorzugt aus einem Schaum. Durch diese Ausgestaltung wird vermieden, dass es bei angetriebenem und damit bewegtem Bodenbearbeitungselement zu einem hörbaren Aneinanderschlagen von Duftelement und Wandung des Hohlraumes kommt. Somit kann ein störendes Klappern oder Abrollgeräusch des Duftelementes innerhalb des Hohlraumes vermieden werden. Bei dem weichelastischen Material kann es sich um ein offenporiges Material handeln, insbesondere um einen Schaum, welches Poren zur Aufnahme von Duftpartikeln und /oder duftenden Flüssigkeiten aufweist. Insbesondere kann das Duftelement auch ein mit einem Duftöl getränkter Schwamm oder dergleichen sein.

Es wird gemäß einer Ausführungsform vorgeschlagen, dass das Duftelement innerhalb des Hohlraums frei beweglich ist, wobei der Hohlraum insbesondere ein Volumen aufweist, welches einem Vielfachen des Volumens des Duftelementes entspricht. Bei dieser Ausgestaltung bewegt sich das Duftelement während des Antriebes des Bodenbearbeitungselementes relativ zu der Wandung des Hohlraums, so dass es zu zusätzlichen Luftverwirbelungen innerhalb des Hohlraums kommt. Dabei wird der Duft des Duftelementes verteilt, wobei der entstehende Luftstrom die Duftmoleküle mitreißt und bei Austreten durch die Hohlraumöffnung an die Umgebungsluft abgibt.

Insbesondere wird vorgeschlagen, dass der Hohlraum ein Volumen aufweist, welches einem Vielfachen des Volumens des Duftelementes entspricht. Beispielsweise kann das Volumen des Hohlraums 5 mal, 10mal, 20 mal, 30 mal, 40 mal bis hin zu 100 mal oder darüber größer sein als das Volumen des Duftelementes, wobei das Duftelement frei innerhalb des Hohlraums beweglich ist. Es bietet sich insbesondere an, dass die Geometrie des Duftelementes an die Geometrie des Hohlraumes angepasst ist, beispielsweise ein kugelförmiges Duftelement innerhalb eines zylindrischen Hohlraumes verlagerbar ist. Selbstverständlich sind jedoch auch andere Geometrien des Duftelementes denkbar, beispielsweise quadratische, rechteckige und/oder polygonale Geometrien. Es kann desWeiteren vorgesehen sein, dass das Duftelement um eine Rotationsachse rotierbar innerhalb des Hohlraums gehalten ist, wobei das Duftelement bei einer Rotation des Bodenbearbeitungselementes relativ zu der Umgebung drehfest bleibt, während der Hohlraum um das Duftelement herum rotiert. Des Weiteren kann das Duftelement insbesondere auch nach der Art eines Speichenrades, Fächers oder dergleichen ausgebildet sein, in Umfangsrichtung oder axiale Richtung des Bodenbearbeitungselementes, wobei bevorzugt gleichzeitig eine Luftverwirbelung innerhalb des Hohlraumes provoziert wird.

Alternativ kann vorgesehen sein, dass das Duftelement drehfest an einer den Hohlraum begrenzenden Wandung angeordnet ist. Bei dieser Ausgestaltung bewegt sich das Duftelement bei Antrieb des Bodenbearbeitungselementes nicht relativ zu der Wandung. Es wird somit ohne weitere Maßnahmen verhindert, dass das Duftelement an eine Wandung des Hohlraums anschlägt, an der Wandung abrollt oder sonstwie ungewünscht in Kontakt mit der Wandung tritt. Somit werden störende Geräusche während des Betriebs des Bodenbearbeitungsgerätes verhindert. Es kann insbesondere vorgesehen sein, dass das Duftelement in einer in dem Hohlraum angeordneten Halterung fixiert ist.

Gemäß einer besonderen Ausführungsform wird vorgeschlagen, dass der Hohlraum durch zwei ineinander liegende und relativ zu einander verlagerbare Wandungen begrenzt ist, wobei eine erste Wandung eine erste Hohlraumöffnung aufweist und eine zweite Wandung eine zweite Hohlraumöffnung aufweist, wobei infolge einer Verlagerung der Wandungen relativ zueinander die erste Hohlraumöffnung mittels der zweiten Wandung und die zweite Hohlraumöffnung mittels der ersten Wandung verschließbar oder öffenbar ist. Gemäß dieser Ausgestaltung verfügt das Bodenbearbeitungselement über zwei ineinander laufende Wandungen, welche jeweils mindestens eine Hohlraumöffnung aufweisen. Besonders bevorzugt weisen die Wandungen jeweils mehrere Hohlraumöffnungen auf, die ein regelmäßiges oder unregelmäßiges Lochmuster innerhalb der Wandungen bilden. Sofern das Bodenbearbeitungselement zylindrisch ausgebildet ist, können die Wandungen beispielsweise koaxiale Hohlzylinder sein, welche relativ zueinander in Umfangsrichtung rotieren. Während des Betriebs des Bodenbearbeitungsgerätes, d. h. bei Antrieb des Bodenbearbeitungselementes, dreht sich eine der Wandungen um die andere, wobei sich die Hohlraumöffnungen regelmäßig oder unregelmäßig überlappen und dabei mit Duftmolekülen angereicherte Luft aus dem Hohlraum ausströmen lassen oder nicht. Während des Stillstandes des Bodenbearbeitungselementes ist die Wahrscheinlichkeit der Überlappung der Hohlraumöffnungen der beiden Wandungen gering zu halten, so das mit Duft beaufschlagte Luft nur während der Bewegung des Bodenbearbeitungselementes austritt. Für einen Nutzer des Bodenbearbeitungsgerätes ist aufgrund des wahrnehmbaren Duftes eine erfolgreich abgeschlossene Reinigung erkennbar. Sofern das Bodenbearbeitungsgerät nicht benutzt wird, d. h. das Bodenbearbeitungselement still steht, sind die Hohlraumöffnungen verschlossen, so dass der Duft des Duftelementes in der Umgebungsluft nicht wahrnehmbar ist.

Gemäß einer alternativen Ausführungsform wird vorgeschlagen, dass der Hohlraumöffnung ein mechanisch betätigbares Ventilelement zugeordnet ist, welches abhängig von dem Betrag einer aufgrund einer Rotation des Bodenbearbeitungselementes auf das Ventilelement wirkenden Zentrifugalkraft in eine die Hohlraumöffnung verschließende Schließstellung und/oder in eine die Hohlraumöffnung freigebende Öffnungsstellung verlagerbar ist. Dabei hängt es von der an dem Ventilelement angreifenden Zentrifugalkraft ab, ob die Hohlraumöffnung geöffnet ist oder nicht. Der Begriff mechanisch betätigbares Ventilelement bezeichnet ein selbsttätig kraftabhängig arbeitendes Ventil und kein manuell von einem Nutzer zu betätigendes Ventil. Dabei liegt die Erkenntnis zugrunde, dass bei einer Rotation des Bodenbearbeitungselementes eine drehzahlabhängige Zentrifugalkraft auf das Ventilelement wirkt. Sobald die Zentrifugalkraft die Schließkraft des Ventilelementes überwiegt, öffnet das Ventilelement für einen Austritt von Luft aus dem Hohlraum. Dabei ist die auf das Ventilelement wirkende Zentrifugalkraft hauptsächlich von der Masse des Ventilelementes und der Drehzahl des Bodenbearbeitungselementes abhängig. Bei Überschreiten einer definierten Mindest-Zentrifugalkraft wird die Hohlraumöffnung von dem Ventilelement frei gegeben. Dabei wird das Ventilelement von der Schließstellung in die Öffnungsstellung verlagert. Insbesondere ist diese Ausführungsform auch für Bodenbearbeitungselemente geeignet, in deren Hohlraum eine Flüssigkeit gespeichert ist, welche durch die Hohlraumöffnung nach außen, beispielsweise auf die Oberfläche des Bodenbearbeitungselements, entweichen soll. In diesem Fall ist das Bodenbearbeitungselement beispielsweise eine von innen befeuchtete Reinigungswalze, welche eine Feuchtreinigung der zu bearbeitenden Fläche ausführt. Durch das ebenfalls in dem Hohlraum befindliche Duftelement kann die Flüssigkeit mit Duftmolekülen versehen werden. Anhand der duftenden Flüssigkeit bzw. auf der zu reinigenden Fläche hinterlassenen Duftmolekülen kann der Nutzer die erfolgte Feuchtreinigung erkennen. In dem Fall einer von innen befeuchtete Reinigungswalze, hängt der Austritt von Flüssigkeit aus dem Hohlraum unter anderem von der Masse der Flüssigkeit, der Masse des Ventilelementes, dem Abstand der Flüssigkeit bzw. des Ventilelementes zu der Rotationsachse und der Drehzahl der Reinigungswalze ab.

Insbesondere wird vorgeschlagen, dass das Ventilelement ausgebildet ist, ab Erreichen einer Mindestdrehzahl des Bodenbearbeitungselementes die Hohlraumöffnung freizugeben. Bei im Übrigen konstanten Parametern ist die Verlagerung des Ventilelementes von der Schließstellung in die Öffnungsstellung, und umgekehrt, ausschließlich von der Drehzahl der Reinigungswalze um die Drehachse abhängig. Dabei ist eine Mindestdrehzahl definiert, bei deren Überschreitung das Ventilelement in die Öffnungsstellung verlagert wird und somit einen Austritt von Flüssigkeit aus dem Hohlraum ermöglicht. Somit kann über die Drehzahleinstellung des Bodenbearbeitungselementes gezielt gesteuert werden, wann die Hohlraumöffnungen freigegeben wird und somit Duftmoleküle aus dem Hohlraum austreten können. Während eines bloßen Transports des Bodenbearbeitungsgerätes, bei welchem sich das Bodenbearbeitungselement in der Regel nicht bewegt, kann der Austritt von Duftmolekülen somit wirksam verhindert werden.

Es wird vorgeschlagen, dass dem Ventilelement ein Rückstellelement, insbesondere ein Federelement, zugeordnet ist und/oder das Ventilelement als Rückstellelement ausgebildet ist, wobei die Rückstellkraft des Rückstellelementes entgegen der Zentrifugalkraft in Richtung der Schließstellung wirkt. Die Rückstellkraft des Rückstellelementes wirkt der an dem Ventilelement angreifenden Zentrifugalkraft entgegen. Das Rückstellelement kann separat zu dem Ventilelement ausgebildet sein, beispielsweise in Form einer separaten Druckfeder, Zugfeder, Drehfeder oder dergleichen. Alternativ kann das Ventilelement selbst als Rückstellelement ausgebildet sein oder das Rückstellelement als integralen Bestandteil aufweisen. Beispielsweise kann das Ventilelement aus einem elastischen Material gebildet sein, welches aufgrund der Zentrifugalkraft zumindest teilweise verlagerbar ist. Beispielsweise kann das Ventilelement ein Filmscharnier aufweisen oder im Randbereich der Hohlraumöffnung elastisch ausgebildet sein, so dass eine Verlagerung aufgrund der angreifenden Zentrifugalkraft möglich ist. Das Rückstellelement ist dabei jeweils so auszulegen, dass die Rückstellkraft bei einer definierten Drehzahl des Bodenbearbeitungselementes geringer ist als die angreifende Zentrifugalkraft, so dass das Ventilelement bei Überschreiten der Mindestdrehzahl öffnet.

Es kann vorgesehen sein, dass das Ventilelement mindestens ein schwenkbeweglich an einer Wandlung des Hohlraums angeordnetes Verschlusselement aufweist. Dieses Verschlusselement kann beispielsweise eine schwenkbewegliche Ventilklappe sein, welche an dem die Hohlraumöffnung begrenzenden Randbereich der Wandung angeordnet ist. Das schwenkbewegliche Verschlusselement kann entweder ein separates, an dem Hohlkörper befestigtes Verschlusselement sein, oder integral mit dem Hohlkörper ausgebildet sein, beispielsweise als Filmscharnier, elastischer Randbereich oder ähnliches.

Das Ventilelement kann des Weiteren ein linear beweglich, insbesondere schiebebeweglich, an dem Bodenbearbeitungselement angeordnetes Verschlusselement sein. Des Weiteren kann das Ventilelement ein elastisch ausgebildeter, die Hohlraumöffnung begrenzender Randbereich der Wandung des Hohlraumes sein. Gemäß dieser Ausgestaltung weist die Wandung zumindest im Randbereich der Hohlraumöffnung ein elastisches Material auf, so dass sich der Randbereich aufgrund der angreifenden Zentrifugalkraft nach radial außen verlagert und die Hohlraumöffnung vergrößert wird. Bezogen auf die Schließstellung ist der elastische Randbereich vorteilhaft so ausgebildet, dass in der Schließstellung einander überlappende Teilbereiche der Wandung vorgesehen sind. In der Öffnungsstellung sind die Teilbereiche der Wandung voneinander beabstandet und ist die Hohlraumöffnung somit frei gegeben.

Der Hohlraum kann insgesamt oder zumindest teilweise eine elastische Membran aufweisen, wobei mindestens eine Hohlraumöffnung in der Schließstellung zusammen gezogen und in der Öffnungsstellung auseinander gedrückt ist. Der Hohlraum kann somit aus einer Membran gebildet sein, welche beispielsweise nadelstichförmige Hohlraumöffnungen aufweist, die sich abhängig von dem Betrag der angreifenden Zentrifugalkraft mehr oder weniger öffnen und somit einen Strömungsweg für aus dem Hohlraum ausströmende Luft und Duftmoleküle freigeben. Die Hohlraumöffnungen sind somit in der Schließstellung punktförmig zusammengezogen und in der Öffnungsstellung auseinandergedrückt. Es empfiehlt sich dabei, für die Membran ein Material mit einem Elastizitätsmodul auszuwählen, der ein schnelles Öffnen und Schließen der Hohlraumöffnung in Abhängigkeit von einer Änderung des Betrages der Zentrifugalkraft erlaubt. Insbesondere eignen sich gummiartige Materialien.

Das erfindungsgemäße Bodenbearbeitungsgerät kann ein Feuchtreinigungsgerät, ein Trockenreinigungsgerät oder auch ein anderes Bodenbearbeitungsgerät, wie beispielsweise eine Polier- und/oder Schleifeinrichtung sein. Es wird vorgeschlagen, dass die definierte Mindestdrehzahl des Bodenbearbeitungselementes mindestens 150 Umdrehungen pro Minute bis hin zu 3000 Umdrehungen pro Minute beträgt. Die angegebene Mindestdrehzahl von 150 Umdrehungen pro Minute hebt sich deutlich von einer nur zufälligen Rotation des Bodenbearbeitungselementes während eines Transports des Bodenbearbeitungsgerätes ab. Ebenso übersteigt diese Mindestdrehzahl auch die Drehzahl des Bodenbearbeitungselementes bei einem bloßen Verschieben des Bodenbearbeitungsgerätes über die zu bearbeitende Fläche. Insbesondere entspricht der angegebene Drehzahlbereich üblichen Rotationsdrehzahlen des Bodenbearbeitungselementes während eines Bearbeitungsvorgangs zum Reinigen einer Fläche.

Schließlich wird vorgeschlagen, dass das Bodenbearbeitungselement radial nach außen weisende Borstenelemente aufweist oder mit einem Schwammkörper ummantelt ist. Zusätzlich oder alternativ kann vorgesehen sein, dass das Bodenbearbeitungselement und/oder der Schwammkörper mit einem Reinigungstuch, insbesondere einem Mikrofasertuch, ummantelt ist. Beispielsweise kann es sich um ein textiles Reinigungstuch handeln, in welchem der von der zu bearbeitenden Fläche gelöste Schmutz gebunden wird. Als besonders vorteilhaft erweist sich in diesem Zusammenhang die Ausbildung des Reinigungstuches als Mikrofasertuch.

### Kurze Beschreibung der Zeichnungen

Im Folgenden wird die Erfindung anhand von Ausführungsbeispielen näher erläutert. Es zeigen:
- Fig. 1: ein Bodenbearbeitungsgerät in perspektivischer Ansicht,
- Fig. 2: einen Querschnitt durch ein Bodenbearbeitungselement des Bodenbearbeitungsgerätes gemäß einer ersten Ausführungsform,
- Fig. 3: einen Querschnitt durch ein Bodenbearbeitungselement gemäß einer zweiten Ausführungsform,
- Fig. 4: einen Querschnitt durch ein Bodenbearbeitungselement gemäß einer dritten Ausführungsform zu einem ersten Zeitpunkt während der Rotation des Bodenbearbeitungselementes,
- Fig. 5: das Bodenbearbeitungselement gemäß Fig. 4 zu einem zweiten Zeitpunkt während der Rotation,
- Fig. 6: eine Unteransicht eines Bodenbearbeitungsgerätes mit einem Bodenbearbeitungselement gemäß einer vierten Ausführungsform,
- Fig. 7: einen vergrößerten Teilbereich des Bodenbearbeitungselementes gemäß Fig. 6,
- Fig. 8: einen Querschnitt durch den vergrößerten Teilbereich des Bodenbearbeitungselementes gemäß Fig. 7 mit einem geschlossenen Ventilelement,
- Fig. 9: den Teilbereich des Bodenbearbeitungselementes gemäß Fig. 8 mit geöffnetem Ventilelement.

### Beschreibung der Ausführungsformen

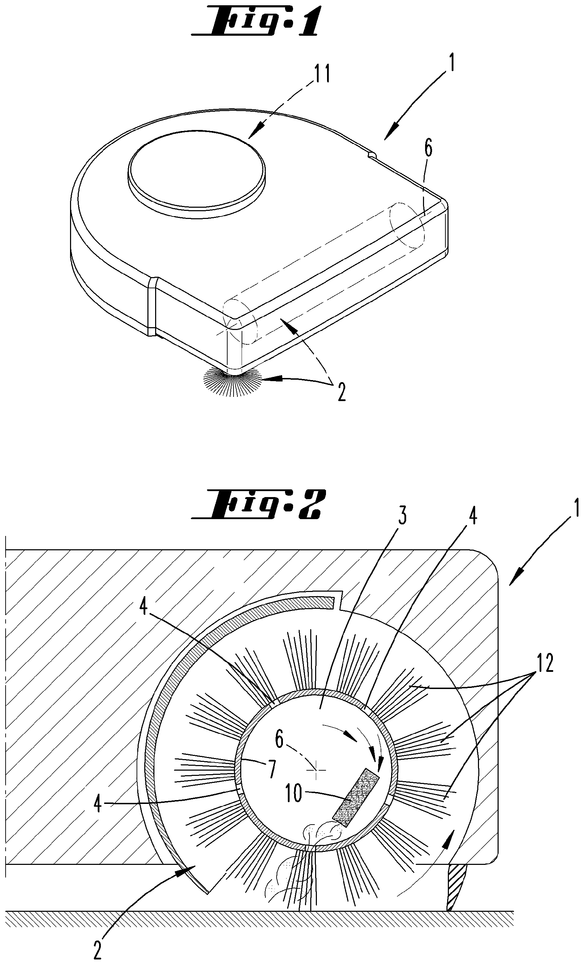

Figur 1 zeigt ein Bodenbearbeitungsgerät 1, welches hier als sich selbstständig fortbewegender Saugroboter ausgebildet ist. Das Bodenbearbeitungsgerät 1 verfügt in üblicher Art und Weise über eine hier nicht näher dargestellte Motor-Gebläse-Einheit sowie eine Sauggutkammer zur Aufnahme von Staub und Schmutz. Des Weiteren verfügt das Bodenbearbeitungsgerät 1 über eine Abstandsmesseinrichtung 11, mittels welcher Abstände zu Hindernissen innerhalb der Umgebung des Bodenbearbeitungsgerätes 1 gemessen werden können. Die Abstandsmesseinrichtung 11 liegt hier innerhalb des Gehäuses des Bodenbearbeitungsgerätes 1 und ist nach der Art einer Triangulationsmesseinrichtung ausgebildet. Diese Triangulationsmesseinrichtung verfügt über eine Laserdiode und eine entsprechende Optik, um den von der Laserdiode emittierten Lichtstrahl aus dem Bodenbearbeitungsgerät 1 heraus zu führen. Dieser Lichtstrahl trifft dann innerhalb der Umgebung des Bodenbearbeitungsgerätes 1 auf Hindernisse und wird dort reflektiert und/oder gestreut. Das reflektierte bzw. gestreute Licht trifft auf einen Sensor der Abstandmesseinrichtung 11 und kann dort in Bezug auf einen Abstand der reflektierenden bzw. streuenden Hindernisse ausgewertet werden.

Das Bodenbearbeitungsgerät 1 verfügt des Weiteren über mehrere Bodenbearbeitungselemente 2, zu welchen hier einerseits eine seitlich neben dem Gehäuse des Bodenbearbeitungsgerätes 1 hervorstehende Seitenbürste sowie eine unterhalb des Bodenbearbeitungsgerätes 1 angeordnete Reinigungswalze gehören. Das als Reinigungswalze ausgebildete Bodenbearbeitungselement 2 ist hier ein zylindrischer Körper, welcher einen zylindrischen Hohlraum 3 aufweist. Das Bodenbearbeitungselement 2 rotiert während eines Reinigungsbetriebs des Bodenbearbeitungsgerätes 1 um eine Drehachse 6, welche während eines üblichen Reinigungsbetriebs des Bodenbearbeitungsgerätes 1 im Wesentlichen parallel zu einer zu reinigenden Fläche orientiert ist.

Figur 2 zeigt einen Querschnitt des Bodenbearbeitungselementes 2 quer zu der Längserstreckung der Drehachse 6. Der Hohlraum 3 ist von einer Wandung 7 begrenzt, welche mehrere Hohlraumöffnungen 4 aufweist, die den Hohlraum 3 des Bodenbearbeitungselementes 2 mit der Umgebungsluft verbinden. Innerhalb des Hohlraums 3 ist ein Duftelement 10 angeordnet, welches frei innerhalb des Hohlraums 3 verlagerbar ist. Das Duftelement 10 ist hier beispielsweise ein Schaumkörper, in dessen Poren ein Duftmoleküle aufweisendes Öl aufgenommen ist. Das Duftelement 10 weist hier beispielsweise eine eckige Querschnittsform auf. Die Härte des Duftelementes 10 ist geringer als die Härte der Wandung 7 des Bodenbearbeitungselementes 2, so dass es bei einer Rotation des Bodenbearbeitungselementes 2 nicht zu einem hörbaren Anschlagen des Duftelementes 10 an der Wandung 7 des Hohlraumes 3 kommt. Das spezifische Gewicht des Duftelementes 10 ist ebenfalls geringer als das der Wandung 7. Die in dem Duftelement 10 aufgenommenen Duftmoleküle können mit einem bei der Rotation des Bodenbearbeitungselementes 2 erzeugten Luftstrom durch die Hohlraumöffnung 4 in die Umgebung entweichen. Der erzeugte Luftstrom reißt die Duftmoleküle mit und verteilt diese in der Luft. Die Wahrnehmbarkeit des Duftes außerhalb des Bodenbearbeitungselementes 2 ist während der Rotation des Bodenbearbeitungselementes 2 größer als während des Stillstandes des Bodenbearbeitungselementes 2. Somit ist die Wahrnehmung des Duftes an den Betrieb des Bodenbearbeitungsgerätes 1 gekoppelt. Ein Nutzer kann somit anhand des innerhalb der Umgebung wahrnehmbaren Duftes erkennen, dass das Bodenbearbeitungsgerät 1 kürzlich eine Bodenbearbeitung wahrgenommen hat oder derzeit noch eine Bodenbearbeitung wahrnimmt. Die Reinigungsleistung des Bodenbearbeitungsgerätes 1 ist somit durch den Nutzer erkennbar. Sofern in der Umgebung des Bodenbearbeitungsgerätes 1 Duft wahrnehmbar ist, deutet dies auf einen Betrieb des Bodenbearbeitungsgerätes 1 hin. Sofern trotz eines geplanten Einsatzes des Bodenbearbeitungsgerätes 1 kein Duft wahrnehmbar ist, erhält der Nutzer aufgrund des fehlenden Duftes eine Information über entweder einen Betriebsfehler des Bodenbearbeitungsgerätes 1 oder ein Ende der Lebensdauer des Duftelementes 10. Das Bodenbearbeitungselement 2 weist an der Außenseite der Wandung 7 eine Vielzahl von Borstenelementen 12 auf, welche hier nach der Art von Borstenbüscheln mit jeweils einer Mehrzahl von Borsten ausgebildet sind. Die Borstenelemente 12 dienen während des Betriebs des Bodenbearbeitungsgerätes 1 einer mechanischen Einwirkung auf die zu reinigende Fläche. Die Lebensdauer der Borstenelemente 12, als Verschleißteile des Bodenbearbeitungsgerätes 1, kann vorteilhaft an die Lebensdauer des Duftelementes 10 gekoppelt sein. Das Bodenbearbeitungselement 2, insbesondere dessen Borstenelemente 12, nutzt sich mit zunehmender Betriebsdauer des Bodenbearbeitungsgerätes 1 ab. Der Duft des Duftelementes 10 wird dabei als Indikator einen notwendigen Wechsel des Bodenbearbeitungselementes 2 bzw. der Borstenelemente 12 genutzt. Sobald der Nutzer das Bodenbearbeitungselement 2 mitsamt den Borstenelementen 12 und dem Duftelement 10 gegen ein neues Bodenbearbeitungselement 2 austauscht, wird während des Betriebs des Bodenbearbeitungsgerätes 1 wieder Duft an die Umgebung abgegeben, so dass der Betrieb des Bodenbearbeitungsgerätes 1 wiederum über den Duft wahrnehmbar ist.

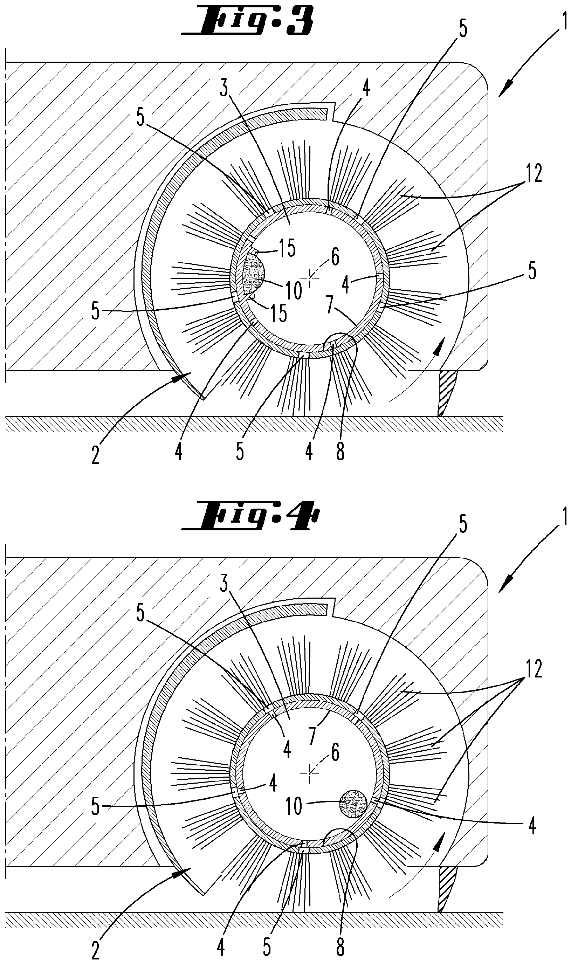

Figur 3 zeigt eine weitere Ausführungsform eines Bodenbearbeitungselementes 2. Innerhalb des Hohlraums 3 dieses Bodenbearbeitungselementes 2 ist ein Duftelement 10 drehfest an der den Hohlraum 3 begrenzenden Wandung 7 angeordnet. Zur Befestigung des Duftelementes 10 dienen hier Befestigungsmittel 15, die das Duftelement 10 an der Wandung 7 halten. Während der Rotation des Bodenbearbeitungselementes 2 dreht sich das Duftelement 10 somit mit der Wandung 7 mit. Entsprechend kommt es nicht zu einer relativen Verlagerung des Duftelementes 10 innerhalb des Hohlraums 3. Die Befestigungsmittel 15 können eine Klemmverbindung oder Rastverbindung zur Verfügung stellen. Des Weiteren ist es aber auch möglich, dass das Duftelement 10 permanent an der Wandung 7 des Bodenbearbeitungselementes 2 befestigt ist, beispielsweise angeklebt ist. Sofern das Bodenbearbeitungselement 2 so ausgestaltet ist, dass das Duftelement 10 getauscht werden kann, beispielsweise über eine Stirnseite des zylindrisch ausgebildeten Bodenbearbeitungselementes 2, empfiehlt es sich, dass die Befestigungsmittel 15 das Duftelement 10 reversibel befestigen.

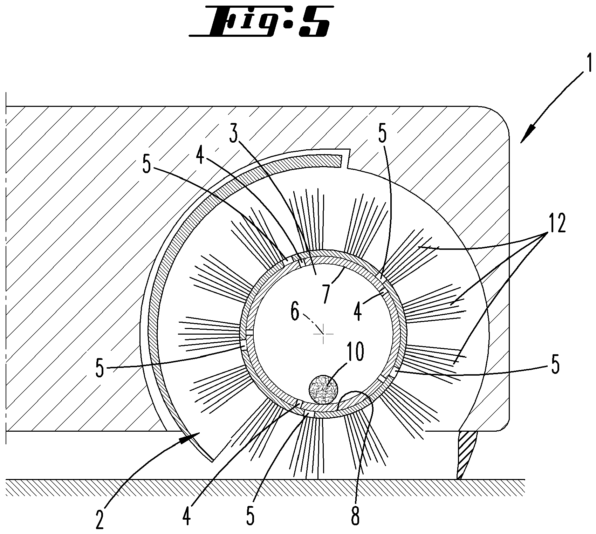

Die Figuren 4 und 5 zeigen eine weitere Ausführungsform eines Bodenbearbeitungselementes 2, welches zwei relativ zueinander rotierbare Wandungen 7,8 aufweist. Eine erste, den Hohlraum 3 begrenzende Wandung 7 ist dabei relativ zu einer äußeren zweiten Wandung 8, an welcher außenseitig Borstenelemente 12 angeordnet sind, rotierbar. Die erste Wandung 7 und die zweite Wandung 8 weisen jeweils eine Mehrzahl von Hohlraumöffnungen 4 bzw. 5 auf. Die Wandungen 7,8 weisen jeweils die Form eines Hohlzylinders auf, wobei die erste Wandung 7 kozentrisch innerhalb der zweiten Wandung 8 angeordnet ist. Während eines Betriebs des Bodenbearbeitungsgerätes 1 dreht sich die erste, innere Wandung 7 relativ zu der äußeren, zweiten Wandung 8 um die Drehachse 6, dabei ändert sich die Position der an den Wandungen 7,8 ausgebildeten Hohlraumöffnungen 4,5 relativ zu einander. Es entstehen Rotationsstellungen, in welchen die Hohlraumöffnungen 4,5 durch Teilbereiche der jeweils anderen Wandung 7,8 überdeckt sind, und Rotationsstellungen, in welchen korrespondierende Hohlraumöffnungen 4,5 der Wandungen 7,8 übereinander liegen und somit einen Strömungsweg zwischen dem Hohlraum 3 des Bodenbearbeitungselementes 2 und der Umgebung frei geben. Während der Rotation des Bodenbearbeitungselementes 2 überlappen sich die Hohlraumöffnungen 4,5 somit regelmäßig oder unregelmäßig und lassen die Duftmoleküle passieren. Im Stillstand des Bodenbearbeitungselementes 2 ist die Wahrscheinlichkeit der Überlappung der Hohlraumöffnungen 4,5 gering zu halten, beispielsweise mittels einer definierten Ruhestellung des Bodenbearbeitungselementes 2, so dass ein Austritt von Duftmolekülen aus dem Hohlraum 3 des Bodenbearbeitungselementes 2 verhindert ist. Das Bodenbearbeitungsgerät 1 gibt den Duft somit nur während des Betriebs des Bodenbearbeitungsgerätes 1 ab, ansonsten ist dieser nicht wahrnehmbar. Der Nutzer erhält somit eine eindeutige Information darüber, ob das Bodenbearbeitungsgerät 1 in Betrieb ist bzw. war, oder nicht. In den Figuren 4 und 5 sind verschiedene Rotationsstellungen der Wandungen 7,8 des Bodenbearbeitungselementes 2 dargestellt. Figur 4 zeigt dabei eine Rotationsstellung, bei welcher sich die korrespondierenden Hohlraumöffnungen 4,5 der ersten Wandung 7 und zweiten Wandung 8 überlappen und somit einen Strömungsweg von dem Hohlraum 3 in die Umgebung frei geben. Figur 5 zeigt demgegenüber eine Rotationsstellung des Bodenbearbeitungselementes 2, bei welcher die Hohlraumöffnungen 4 der ersten Wandung 7 durch die zweite Wandung 8 überdeckt sind bzw. die Hohlraumöffnungen 5 der zweiten Wandung 8 durch die erste Wandung 7 verdeckt sind. Der Strömungsweg von dem Hohlraum 3 an die Umgebung ist somit versperrt.

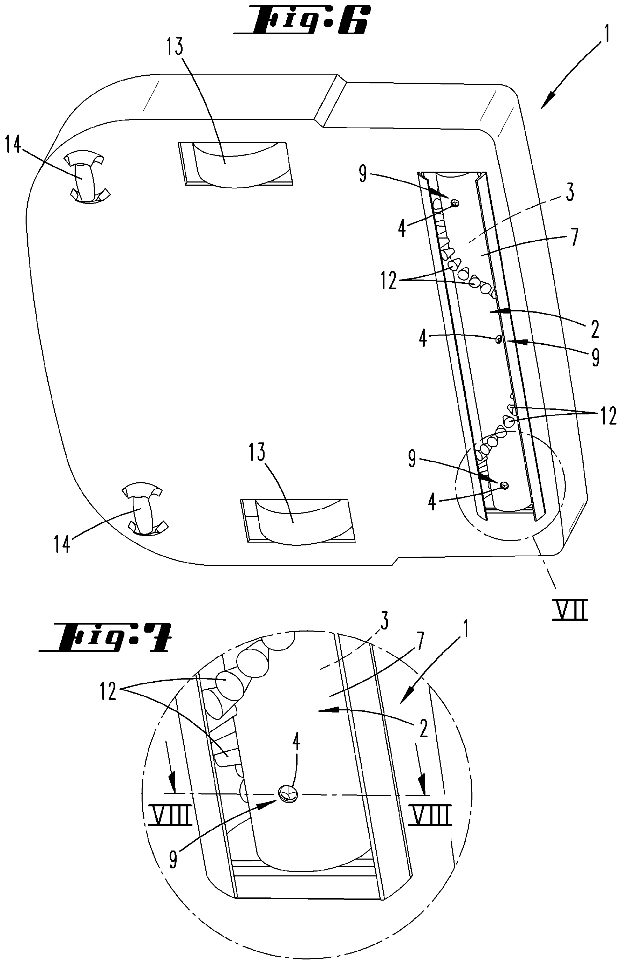

Die Figuren 6 bis 9 zeigen eine weitere Ausführungsform eines erfindungsgemäßen Bodenbearbeitungsgerätes 1. Das Bodenbearbeitungsgerät 1 weist in übriger Art und Weise zwei elektromotorisch angetriebene Räder 13 auf, mit welchen sich das Bodenbearbeitungsgerät 1 auf der zu reinigenden Fläche fortbewegt. Des Weiteren verfügt das dargestellte Bodenbearbeitungsgerät 1 unterseitig über zwei Stützrollen 14. Das Bodenbearbeitungselement 2 ist hier ebenfalls als eine mit Borstenelementen 12 versehene Reinigungswalze ausgebildet. Eine Wandung 7 des Bodenbearbeitungselementes 2 begrenzt wiederum einen innenliegenden, zylindrischen Hohlraum 3 des Bodenbearbeitungselementes 2. Die Wandung 7 weist mehrere Hohlraumöffnungen 4 auf, welche jeweils über ein Ventilelement 9 verfügen. Das Ventilelement 9 ist mechanisch betätigbar und kann abhängig von einer angreifenden Zentrifugalkraft bei Rotation des Bodenbearbeitungselementes 2 entweder die Hohlraumöffnung 4 verschließen oder frei geben. Das Ventilelement 9 ist durch einen elastisch ausgebildeten Randbereich der Wandung 7 gebildet. Hier besteht das Ventilelement 9 beispielsweise aus einem Gummimaterial, welches sich in Abhängigkeit von der dem Material innewohnenden Rückstellkraft und dem Betrag der Zentrifugalkraft in Richtung einer Schließstellung bzw. einer Öffnungstellung verlagern kann. Durch die von radial innen an dem Ventilelement 9 angreifende Zentrifugalkraft kommt es zu einer elastischen Verformung der Randbereiche der Hohlraumöffnung 4 nach radial außen (bezogen auf die Drehachse 6), so dass die Hohlraumöffnung 4 zum Austritt einer Luftströmung aus dem Hohlraum 3 freigegeben wird. Sobald die Zentrifugalkraft aufgrund einer Verringerung der Drehzahl des Bodenbearbeitungselementes 2 unter eine definierte Mindestdrehzahl sinkt, überwiegt die Rückstellkraft des Ventilelementes 9, so dass das Ventilelement 9 wieder in die Schließstellung verlagert wird. Das Ventilelement 9 ist hier einteilig mit den übrigen Teilbereichen der Wandung 7 ausgebildet. Beispielsweise ist das Ventilelement 9 durch Spritzguss an die Wandung 7 angespritzt.

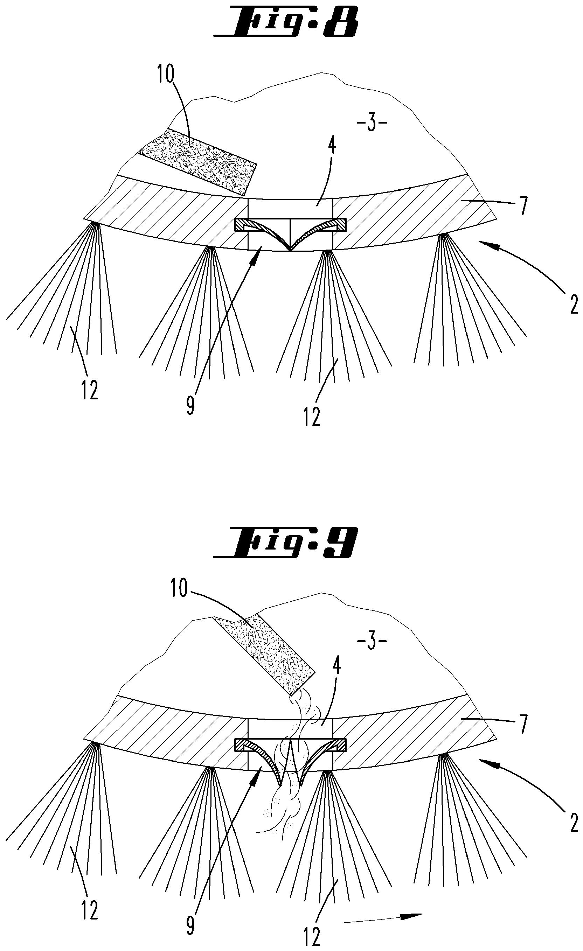

Figur 7 zeigt einen vergrößerten Teilbereich des Bodenbearbeitungselementes 2 mit verschlossenem Ventilelement 9. Figur 8 zeigt einen Querschnitt des Ventilelementes 9 senkrecht zu der Längserstreckung der Drehachse 6.

Figur 9 zeigt schließlich in einem Querschnitt das geöffnete Ventilelement 9, welches aufgrund der bei der Rotation des Bodenbearbeitungselementes 2 an dem Ventilelement 9 angreifenden Zentrifugalkraft nach radial außen geöffnet ist, so dass von dem Duftelement 10 gelöste Duftmoleküle durch die Hohlraumöffnung 4 in die Umgebung des Bodenbearbeitungsgerätes 1 gelangen können.

### Liste der Bezugszeichen

- 1: Bodenbearbeitungsgerät
- 2: Bodenbearbeitungselement
- 3: Hohlraum
- 4: Hohlraumöffnung
- 5: Hohlraumöffnung
- 6: Drehachse
- 7: Wandung
- 8: Wandung
- 9: Ventilelement
- 10: Duftelement
- 11: Abstandsmesseinrichtung
- 12: Borstenelement
- 13: Rad
- 14: Stützrolle
- 15: Befestigungsmittel

## Patentansprüche

1. Bodenbearbeitungsgerät (1) mit einem motorisch angetriebenen Bodenbearbeitungselement (2) zum Bearbeiten einer zu bearbeitenden Fläche, wobei das Bodenbearbeitungselement (2) einen Hohlraum (3) mit einer den Hohlraum (3) mit der Umgebungsluft verbindenden Hohlraumöffnung (4, 5) aufweist, **dadurch gekennzeichnet, dass** in dem Hohlraum (3) ein als Festkörper ausgebildetes Duftelement (10) angeordnet ist, wobei der Hohlraum (3) ein Volumen aufweist, welches einem Vielfachen des Volumens des Duftelementes (10) entspricht.

2. Bodenbearbeitungsgerät (1) nach Anspruch 1, **dadurch gekennzeichnet, dass** das Bodenbearbeitungselement (2) eine um eine Drehachse (6) rotierbare Reinigungswalze ist.

3. Bodenbearbeitungsgerät (1) nach Anspruch 1 oder 2, **dadurch gekennzeichnet, dass** eine Lebensdauer des Duftelementes (10) im Wesentlichen einer Lebensdauer eines Verschleißteils des Bodenbearbeitungsgerätes (1) entspricht, insbesondere einer Lebensdauer von an dem Bodenbearbeitungselement (2) angeordneten Borstenelementen (12).

4. Bodenbearbeitungsgerät (1) nach einem der vorhergehenden Ansprüche, **dadurch gekennzeichnet, dass** das Material des Duftelementes (10) eine geringere Härte aufweist als das Material einer den Hohlraum (3) begrenzenden Wandung (7), wobei das Duftelement (10) insbesondere aus einem weichelastischen Material hergestellt ist, insbesondere aus einem Schaum.

5. Bodenbearbeitungsgerät (1) nach einem der Ansprüche 1 bis 4, **dadurch gekennzeichnet, dass** das Duftelement (10) innerhalb des Hohlraums (3) frei beweglich ist.

6. Bodenbearbeitungsgerät (1) nach einem der Ansprüche 1 bis 4, **dadurch gekennzeichnet, dass** das Duftelement (10) drehfest an einer den Hohlraum (3) begrenzenden Wandung (7) angeordnet ist.

7. Bodenbearbeitungsgerät (1) nach einem der Ansprüche 1 bis 6, **dadurch gekennzeichnet, dass** der Hohlraum (3) durch zwei ineinander liegende und relativ zu einander verlagerbare Wandungen (7, 8) begrenzt ist, wobei eine erste Wandung (7) eine erste Hohlraumöffnung (4) aufweist und eine zweite Wandung (8) eine zweite Hohlraumöffnung (5) aufweist, wobei infolge einer Verlagerung der Wandungen (7, 8) relativ zueinander die erste Hohlraumöffnung (4) mittels der zweiten Wandung (8) und die zweite Hohlraumöffnung (5) mittels der ersten Wandung (5) verschließbar oder öffenbar ist.

8. Bodenbearbeitungsgerät (1) nach einem der Ansprüche 1 bis 6, **dadurch gekennzeichnet, dass** der Hohlraumöffnung (4, 5) ein mechanisch betätigbares Ventilelement (9) zugeordnet ist, welches abhängig von dem Betrag einer aufgrund einer Rotation des Bodenbearbeitungselementes (1) auf das Ventilelement (9) wirkenden Zentrifugalkraft in eine die Hohlraumöffnung (4, 5) verschließende Schließstellung und/oder in eine die Hohlraumöffnung (4, 5) freigebende Öffnungsstellung verlagerbar ist.

9. Bodenbearbeitungsgerät (1) nach Anspruch 8, **dadurch gekennzeichnet, dass** das Ventilelement (9) ausgebildet ist, ab Erreichen einer Mindestdrehzahl des Bodenbearbeitungselementes (2) die Hohlraumöffnung (4, 5) freizugeben.

10. Bodenbearbeitungsgerät (1) nach Anspruch 8 oder 9, **dadurch gekennzeichnet, dass** dem Ventilelement (9) ein Rückstellelement, insbesondere ein Federelement, zugeordnet ist und/oder das Ventilelement (9) als Rückstellelement ausgebildet ist, wobei die Rückstellkraft des Rückstellelementes entgegen der Zentrifugalkraft in Richtung der Schließstellung wirkt.

## Claims

1. A floor processing device (1) with a motor-driven floor processing element (2) for processing a floor to be processed, wherein the floor processing element (2) has a hollow space (3) with a hollow space opening (4, 5) that joins the hollow space (3) with the ambient air, **characterized in that** a fragrance element (10), which is designed as a solid body, is arranged in the hollow space (3), wherein the hollow space (3) has a volume corresponding to a multiple of the volume of the fragrance element (10).

2. The floor processing device (1) according to claim 1, **characterized in that** the floor processing device (2) is a cleaning roller that can rotate around a rotational axis (6).

3. The floor processing device (1) according to claim 1 or 2, **characterized in that** the service life of the fragrance element (10) essentially corresponds to a service life of a wear part of the floor processing device (1), in particular to a service life of bristle elements (12) arranged on the floor processing element (2).

4. The floor processing device (1) according to one of the preceding claims, **characterized in that** the material of the fragrance element (10) has a lower hardness than the material of a wall (7) bordering the hollow space (3), wherein the fragrance element (10) is in particular made out of a soft elastic material, in particular out of a foam.

5. The floor processing device (1) according to one of claims 1 to 4, **characterized in that** the fragrance element (10) can move freely inside of the hollow space (3).

6. The floor processing device (1) according to one of claims 1 to 4, **characterized in that** the fragrance element (10) is non-rotatably arranged on a wall (7) bordering the hollow space (3).

7. The floor processing device (1) according to one of claims 1 to 6, **characterized in that** the hollow space (3) is bordered by two walls (7, 8) that lie one inside the other and can be displaced relative to each other, wherein a first wall (7) has a first hollow space opening (4), and a second wall (8) has a second hollow space opening (5), wherein a displacement of the walls (7, 8) relative to each other makes it possible for the first hollow space opening (4) to be closed or opened by the second wall (8) and the second hollow space opening (5) by the first wall (5).

8. The floor processing device (1) according to one of claims 1 to 6, **characterized in that** the hollow space opening (4, 5) has allocated to it a mechanically activatable valve element (9), which depending on the level of centrifugal force acting on the valve element (9) due to a rotation of the floor processing element (1) can be displaced into a closing position that closes the hollow space opening (4, 5) and/or into an opening position that releases the hollow space opening(4, 5).

9. The floor processing device (1) according to claim 8, **characterized in that** the valve element (9) is designed to release the hollow space opening (4, 5) once a minimum speed of the floor processing element (2) has been reached.

10. The floor processing device (1) according to claim 8 or 9, **characterized in that** the valve element (9) has a restoring element, in particular a spring element, allocated to it, and/or that the valve element (9) is designed as a restoring element, wherein the restoring force of the restoring element acts opposite the centrifugal force in the direction of the closed position.

## Revendications

1. Dispositif de traitement du sol (1) avec un élément de traitement du sol (2) entrainé par moteur pour le traitement d'une surface à traiter, dans lequel l'élément de traitement du sol (2) présente une cavité (3) avec une ouverture de cavité (4, 5) reliant la cavité (3) à l'air environnant, **caractérisé en ce que** dans la cavité (3) est agencé un élément odorant (10) réalisé sous forme de corps solide, la cavité (3) présentant un volume qui correspond à un multiple du volume de l'élément odorant (10).

2. Dispositif de traitement du sol (1) selon la revendication 1, **caractérisé en ce que** l'élément de traitement du sol (2) est un rouleau de nettoyage pouvant tourner autour d'un axe de rotation (6).

3. Dispositif de traitement du sol (1) selon la revendication 1 ou 2, **caractérisé en ce qu'**une durée de vie de l'élément odorant (10) correspond sensiblement à une durée de vie d'une pièce d'usure du dispositif de traitement du sol (1), en particulier une durée de vie d'éléments de poils (12) joints à l'élément de traitement du sol (2).

4. Dispositif de traitement du sol (1) selon l'une des revendications précédentes, **caractérisé en ce que** le matériau de l'élément odorant (10) présente une dureté inférieure à celle du matériau d'une paroi (7) délimitant la cavité (3), l'élément odorant (10) étant réalisé en particulier en un matériau élastique souple, en particulier en une mousse.

5. Dispositif de traitement du sol (1) selon l'une des revendications 1 à 4, **caractérisé en ce que** l'élément odorant (10) est librement mobile dans la cavité (3).

6. Dispositif de traitement du sol (1) selon l'une des revendications 1 à 4, **caractérisé en ce que** l'élément odorant (10) est agencé de manière fixe en rotation à une paroi (7) délimitant la cavité (3).

7. Dispositif de traitement du sol (1) selon l'une des revendications 1 à 6, **caractérisé en ce que** la cavité (3) est délimitée par deux parois (7, 8) disposées l'une dans l'autre et pouvant être déplacées l'une par rapport à l'autre, dans lequel une première paroi (7) présente une première ouverture de cavité (4) et une deuxième paroi (8) présente une deuxième ouverture de cavité (5), dans lequel, par suite d'un déplacement des parois (7, 8) l'une relativement à l'autre, la première ouverture de cavité (4) peut être fermée ou ouverte au moyen de la deuxième paroi (8) et la deuxième ouverture de cavité (5) peut être fermée ou ouverte au moyen de la première paroi (5).

8. Dispositif de traitement du sol (1) selon l'une des revendications 1 à 6, **caractérisé en ce qu'**un élément de soupape (9) actionnable mécaniquement est associé à l'ouverture de cavité (4, 5), lequel peut être déplacé dans une position fermée fermant l'ouverture de cavité (4, 5) et/ou dans une position ouverte ouvrant l'ouverture de cavité (4, 5), en fonction de la valeur d'une force centrifuge agissant sur l'élément de soupape (9) en raison d'une rotation de l'élément de traitement du sol (1).

9. Dispositif de traitement du sol (1) selon la revendication 8, **caractérisé en ce que** l'élément de soupape (9) est conçu pour libérer l'ouverture de cavité (4, 5) dès qu'une vitesse de rotation minimale de l'élément de traitement du sol (2) est atteinte.

10. Dispositif de traitement du sol (1) selon la revendication 8 ou 9, **caractérisé en ce qu'**un élément de rappel, en particulier un élément élastique, est associé à l'élément de soupape (9) et/ou l'élément de soupape (9) est formé en tant qu'élément de rappel, dans lequel la force de rappel de l'élément de rappel agit en direction de la position fermée à l'encontre de la force centrifuge.
